# EUROPÄISCHE PATENTSCHRIFT

(11) **EP 0 744 391 B1**
(45) Veröffentlichungstag und Bekanntmachung des Hinweises auf die Patenterteilung: **26.09.2001**
(21) Anmeldenummer: 96106286.6
(22) Anmeldetag: 22.04.1996
(51) Int. Cl.: C07B 37/04, C07C 201/12, C07C 45/68, C07C 17/266, C07C 41/30

(54) **Verfahren zur Herstellung von aromatischen Acetylenen mit Palladazyklen als Katalysatoren**
Process for the preparation of aromatic acetyles using palladacyclen as acatalysts
Procédé pour la préparation d'acétylènes aromatiques avec palladacyclen comme catalyseur

(30) Priorität: 27.04.1995 DE 19515444
(43) Veröffentlichungstag der Anmeldung: 27.11.1996
(73) Patentinhaber: Aventis Research & Technologies GmbH & Co. KG, 65926 Frankfurt am Main (DE)
(72) Erfinder: Beller, Matthias, Prof. Dr., 65510 Idstein (DE); Reisinger, Claus Peter, Dr., 85748 Graiching (DE); Herrmann, Wolfgang Anton, Prof. Dr., 85354 Freising (DE)
(74) Vertreter: Ackermann, Joachim, Dr.

(56) Entgegenhaltungen:
- EP-A- 0 688 757
- EP-A- 0 688 779
- J. MOL. CATAL. A: CHEM. (JMCCF2,13811169);96; VOL.108 (2); PP.51-56, ANORGANISCH-CHEMISCHES INSTITUT DER TECHNISCHEN UNIVERSITAET MUENCHEN, LICHTENBERGSTRASSE 4, D-85747 GARCHING;MUNCHEN; GERMANY (DE), XP000603459 HERRMANN W A ET AL: "Coordination chemistry and mechanisms of metal-catalyzed C-C coupling reactions. Part 8. Facile catalytic coupling of aryl bromides with terminal alkynes by phospha-palladacycles"
- BULLETIN OF THE ACADEMY OF SCIENCES OF THE USSR DIVISION OF CHEM ICAL SCIENCE., Bd. 37, Nr. 3, 1988, NEW YORK US, Seiten 507-509, XP002014703 N.A. BUMAGIN ET AL.: "Reactions of terminal acetylenes with aryl iodides catalyzed by palladium complexes under interfacial conditions"

## Beschreibung

Die vorliegende Erfindung betrifft ein neues Verfahren zur Herstellung von aromatischen Acetylenen mit neuartigen Katalysatoren, sogenannten Palladazyklen.

Aromatische Acetylene, insbesondere Tolan-Derivate, haben technische Bedeutung als Struktureinheiten von Flüssigkristallen und Wirkstoffvorprodukte.

Eine häufig angewandte Methode zur Synthese von aromatischen Acetylenen im Labormaßstab ist die palladiumkatalysierte Kupplung, bei der lod-, Bromaromaten und in Ausnahmefällen Chloraromaten mit monosubstituierten Acetylenen in Gegenwart von Palladiumkatalysatoren und generell katalytischen Mengen an Kupfer(I)-iodid als Cokatalysator umgesetzt werden. Beispiele, die diese Methodik beschreiben, findet man in H.A. Dieck, R.F. Heck, J. Organomet. Chem., 93 (1975) 259; R.F. Heck, Palladium Reagents in Synthesis, Academic Press, London 1985.

Trotz der Vielzahl von Veröffentlichungen auf dem Gebiet der Synthese von aromatischen Acetylenen in Gegenwart von Palladiumkatalysatoren, sind bisher keine Beispiele für eine technische Umsetzung der Methodik bekannt. Dies ist darauf zurückzuführen, daß für die beschriebenen Katalysatorsysteme häufig großen Mengen an Palladiumkatalystor und Kupfer(I)-iodid als Cokatalysator - beide zwischen 1 bis 10 mol% - zugesetzt werden, um technisch nutzbare Umsätze zu erzielen. Aufgrund der Komplexibilität der Reaktionsgemische ist kein einfaches Katalysatorrecycling möglich, so daß die Katalysatorkosten in der Regel eine technische Realisierung erschweren.

Aus den genannten Gründen ist es von großem industriellem Interesse bessere, technisch nutzbare, Katalysatorsysteme für die Synthese von aromatischen Acetylenen allgemein und insbesondere für die Verwendung von ökonomisch günstigen Bromaromaten zu finden. Es bestand somit ein großer Bedarf nach einem Verfahren, das die beschriebenen Nachteile vermeidet und aromatische Acetylene in technisch einfacher Weise zugänglich macht.

Diese Aufgabe wird gelöst durch ein Verfahren zur Herstellung von aromatischen Acetylenen der Formel (I)

(I) Ar-C≡C-R^{8a}

worin Ar für 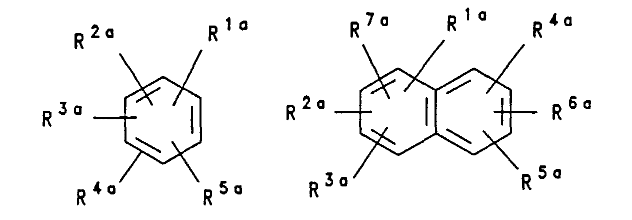 steht und
R^{1a} bis R^{7a} unabhängig voneinander Wasserstoff, C₁-C₈-Alkyl, Alkoxy-(C₁-C₈), Acyloxy-(C₁-C₈), OPhenyl, Phenyl, Fluor, Chlor, Brom, OH, NO₂, CN, COOH, CHO, SO₃H, SO₂R, SOR, NH₂, NHAlkyl-(C₁-C₁₂), N-Alkyl₂-(C₁-C₁₂), C-Hal₃, NHCO-Alkyl-(C₁-C₈), CO-Alkyl-(C₁-C₈), COO-Alkyl-(C₁-C₁₂), CONH₂, CO-Alkyl-(C₁-C₁₂), NHCOH, NCOO-Alkyl-(C₁-C₈), CO-Phenyl, COO-Phenyl, CHCHCO₂-Alkyl-(C₁-C₁₂), CHCHCO₂H, PO-Phenyl₂, PO-Alkyl₂-(C₁-C₈), 5-Ring-Heteroaryl, 6-Ring-Heteroaryl bedeuten, wobei R=(C₁-C₈)Alkyl oder Phenyl ist und R^{8a} für H, C₁-C₁₂-Alkyl, Si(CH₃)₃, CH₂OH, C(CH₃)₂OH oder Ar steht, durch Umsetzung von Halogenaromaten oder Arylsulfonaten der Formel (II)

Ar-X (II)

mit monosubstituierten Acetylenen der Formel (III)

H―C≡C―R^{8a} (III)

worin Ar und R^{8a} die angegebene Bedeutung besitzen und X für Brom oder Chlor oder OSO₂R steht, dadurch gekennzeichnet, daß man als Katalysator eine Palladiumverbindung der allgemeinen Formel (IV) 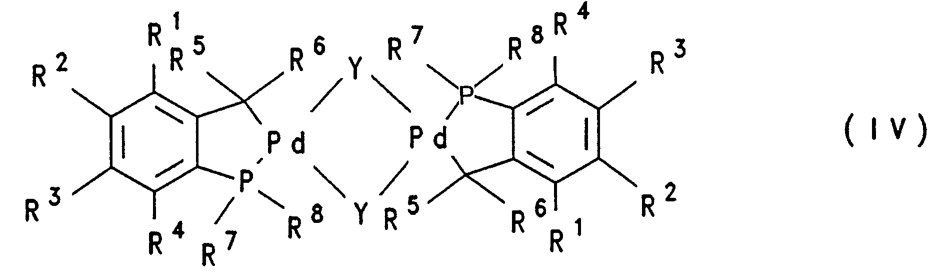 worin
R¹, R², R³, R⁴, R⁵, R⁶ unabhängig voneinander Wasserstoff, (C₁-C₄)-Alkyl, (C₁-C₄)-Alkoxy, Fluor, NH₂, NH-Alkyl-(C₁-C₄), N(Alkyl)₂-(C₁-C₄), CO₂-Alkyl-(C₁-C₄), OCO-Alkyl-(C₁-C₄), oder Phenyl bedeuten,
oder R¹ und R², R² und R³, R³ und R⁴, R⁵ und R⁶ zusammen einen aliphatischen oder aromatischen Ring bilden, und
R⁷, R⁸(C₁-C₈)-Alkyl, (C₃-C₁₂)-Cycloalkyl, substituiertes oder unsubstituiertes Aryl sind und Y ein Anion einer anorganischen oder organischen Säure bedeutet, einsetzt.

Wichtig ist das Verfahren zur Herstellung von Verbindung der Formel (I), worin R^{1a} bis R^{7a} in Formel (I) Wasserstoff, C₁-C₄-Alkyl, Alkoxy-(C₁-C₄), Acyloxy-(C₁-C₄), OPhenyl, Phenyl, Fluor, Chlor, OH, NO₂, CN, COOH, CHO, NH₂, NHAlkyl-(C₁-C₆), N-Alkyl₂-(C₁-C₆), NHCO-Alkyl-(C₁-C₄), CO-Alkyl-(C₁-C₄), COO-Alkyl-(C₁-C₆), CONH₂, CO-Alkyl-(C₁-C₆), NHCOH, NCOO-Alkyl-(C₁-C₄), CHCHCO₂-Alkyl-(C₁-C₆), CHCHCO₂H, 5-Ring-Heteroaryl, 6-Ring-Heteroaryl darstellen.

Von Interesse ist das Verfahren zur Herstellung von Verbindungen der Formel (I) worin 3, insbesondere 4, der Substituenten R^{1a} bis R^{7a} Wasserstoff darstellen und die restlichen Substituenten, die oben angegebene Bedeutung besitzen.

Von großer Bedeutung ist das Verfahren z.B. zur Herstellung der folgenden Verbindungen 1-(4-Nitrophenyl)-2-phenylethin, 1-(4-Acetylphenyl)-2-phenylethin, 2-(6-Methoxynaphthyl)-1-trimethyl-silylethin, 3-(6-Methoxynaphthyl)-1-methylpropargylalkohol, (4-Isobutylphenyl)-1-ethin, 6-Methoxynaphthyl-2-ethin, 4-Hydroxyphenyl-1-ethin, 4-(2-Hydroxyethoxy)phenyl-1-ethin, 1-(4-Chlorphenyl)-2-phenylethin, 1-(4-n-Butylphenyl)-2-phenylethin, 1-(4-Methoxyphenyl)-2-phenylethin, 3-(4-Acetylphenyl)-1-trimethylsiloxy-prop-2-in, 3-(4-Acetylphenyl)-1-tetrahydropyranyl-prop-2-in, 3-(4-Chlorphenyl)-1-trimethylsiloxy-prop-2-in, 3-(4-Chlorphenyl)-1-tetrahydropyranyl-prop-2-in, 1-(2-Pyridyl)-2-phenylethin und 4-(6-Methoxynaphthyl)-1-methyl-but-2-in-1-ol.

In vielen Fällen haben sich Verbindungen der Formel (IV), worin R¹ bis R⁶ Wasserstoff, Alkyl-(C₁-C₄), Phenyl, Cycloalkyl-(C₅-C₈), R⁷ und R⁸ Phenyl, Tolyl, Xylyl, Mesityl, Alkyl-(C₁-C₈) und Cycloalkyl-(C₅-C₈) bedeuten und Y für Acetat, Propionat, Benzoat, Chlorid, Bromid, lodid, Fluorid, Sulfat, Hydrogensulfat, Nitrat, Phosphat, Tetrafluorborat, Tosylat, Mesylat, Acetylacetonat, Hexafluoracetylacetonat oder Pyrazolyl steht, bewährt.

Gut geeignet sind z.B. auch Verbindungen der Formel (IV) worin R¹ bis R⁶ für H, Alkyl, Phenyl und R⁷, R⁸ für Alkyl, Phenyl, Tolyl, Mesityl und Xylyl stehen.

Sehr gute Ergebnisse liefern die Verbindungen:
trans-Di-*µ*-acetato-bis[o-di-tolylphosphino)benzyl]dipalladium(II)
trans-Di-*µ*-chloro-bis[di-o-tolylphosphino)benzyl]dipalladium(II)
trans-Di-*µ*-bromo-bis[o-di-o-tolylphosphino)benzyl]dipalladium(II)
trans-Di-*µ*-iodo-bis[o-(di-o-tolylphosphino)benzyl]dipalladium(II)
trans-Di-*µ*-acetato-bis[o-(dimesitylphosphino)-3,5-dimethylbenzyl]dipalladium(ll)
trans-Di-*µ*-chloro-bis[o-(dimesitylphosphino)-3,5-dimethylbenzyl]dipalladium(II)
trans-Di-*µ*-bromo-bis[o-(dimesitylphosphino)-3,5-dimethylbenzyl]dipalladium(II)
trans-Di-*µ*-iodo-bis[o-(dimesitylphosphino)-3,5-dimethylbenzyl]dipalladium(II)

Als Lösungsmittel finden generell inerte organische Lösungsmittel Verwendung. Bevorzugt werden dipolar aprotische und polare Lösungsmittel, insbesondere alkylierte Amine eingesetzt. Besonders bevorzugt ist als Lösungsmittel Triethylamin.

Da bei der Reaktion Halogenwasserstoff abgespalten wird, ist es vorteilhaft diesen durch Zusatz einer Base abzufangen. Dazu geeignet sind primäre, sekundäre oder tertiäre Amine wie Alkylamine, Dialkylamine, Trialkylamine, die alicyclisch oder offenkettig sein können.

Daneben können Alkali- oder Erdalkalisalze von organischen Verbindungen mit einem pka > 7 verwendet werden.

Die eingesetzten Palladiumkatalysatoren werden in der Regel vor der eigentlichen Reaktion isoliert synthetisiert, sie können jedoch auch in bestimmten Fällen in situ erzeugt werden.

Das Verfahren wird im allgemeinen bei Temperaturen von 20 bis 150°C durchgeführt. Bewährt haben sich Temperaturen von 50 bis 120°C, insbesondere 60 bis 100°C.

Die Synthese der eingesetzten Palladiumkatalystoren erfolgt analog zu dem deutschen Patent DE 44 21 753 C1

Die eingesetzten oder sich bildenden Palladazyklen haben in der Regel dimeren Charakter. Bei bestimmten Verbindungen können jedoch auch monomeren oder polymere Strukturen vorliegen.

Katalysatorsysteme, die im Rahmen der Umsetzung von Arylhalogeniden mit monosubstituierten Acetylenen verwendet werden, sind generell Palladiumverbindungen und in der Regel Kupfer(I)-iodid als Cokatalysator. Obwohl sowohl Palladium(II)- als auch Palladium(O)-Komplexe bei Acetylen-Kupplungen eingesetzt werden, ist es doch allgemein akzeptiert, daß lediglich Palladium-(O)-Verbindungen die eigentlichen Katalysatoren der Reaktion sind. Dabei formuliert man häufig koordinativ ungesättigte 14-Elektronen Palladium(O)-Spezies, welche in der Regel mit schwachen Donorliganden wie Phosphanen stabilisiert werden.

Vor diesem Hintergrund sind die Vorteile der bei den erfindungsgemäßen Verfahren eingesetzten Katalysatoren besonders überraschend.

Die als neue Katalysatorsysteme eingesetzten Palladazyklen zeichnen sich durch sehr große Aktivität und unerwarteterweise damit einhergehende hohe Stabilität aus. Die beschriebenen Katalysatorsysteme sind die bisher aktivsten Katalysatoren für die erfindungsgemäße Umsetzung. So können Turnover-Werte in der Größenordnung von 8000 realisiert werden.

Aufgrund der Katalysatoraktivitäten und -stabilität ist es somit möglich, extrem kleine Mengen an Katalysator zu verwenden, so daß die Katalysatorkosten im Vergleich zu herkömmlichen Acetylen-Kupplungen für den entsprechenden Prozeß nicht kostenlimitierend sind und auf den Cokatalysator Kupfer(I)-iodid gänzlich verzichtet werden kann.

Außerdem bedingt der Einsatz minimaler Mengen an Palladiumkatalysator und der Verzicht an Cokatalysator ökologische Vorteile, da sowohl Abfallprodukte als auch aufwendigeres Katalysatorecycling vermieden werden kann.

Die nachstehenden Beispiele dienen zur Erläuterung des erfindungsgemäßen Verfahrens, ohne es darauf zu beschränken.

### Beispiel 1

2,02 g (10 mmol 4-Bromnitrobenzol und 1,2 g (12 mmol) Phenylacetylen werden mit 10 g (0,1 mol%) trans-Di-*µ*-acetato-bis[o-tolylphosphino)-benzyl]dipalladium(II) in 30 ml Triethylamin 4 Stunden auf 90°C erhitzt. Die Reaktionslösung wird heiß filtriert, um das quantitativ ausgefallene Triethylammoniumbromid abzutrennen, und das Produkt mittels fraktionierter Kristallisation abgetrennt.
Ausbeute: 95 % 1-(4-Nitrophenyl)-2-phenylethin.

### Beispiel 2

2,0 g (10 mmol 4-Bromacetatophenon und 1,2 g (12 mmol) Phenylacetylen werden mit 10 mg (0,1 mol%) trans-Di-*µ*-acetato-bis[o-(di-o-tolylphosphino)benzyl]palladium(II) in 30 ml Triethylamin 4 Stunden auf 90°C erhitzt. Die Reaktionslösung wird heiß filtriert, um das quantitativ ausgefallene Triethylaminammoniumbromid abzutrennen, und das Produkt mittels fraktionierter Kristallisation abgetrennt.
Ausbeute: 96 % (1-(4-Acetylphenyl)-2-phenylethin.

### Beispiel 3 bis 13:

2,02 g (10 mmol) 4-Bromnitrobenzol und 1,2 g (12 mol) Phenylacetylen werden mit 10 mg (0,1 mol%) Katalysator 1 in 30 ml Triethylamin auf 90°C erhitzt. Nach 4 h unter Rückfluß wird das ausgefallene Triethylammoniumbromid heiß abfiltriert, wonach unmittelbar das Produkt im Filtrat kristallisiert. Man erhält 1,73 g an 97 %-igem 1-(4-Nitrophenyl)-2-phenylethin (entspricht 80 % d.Th.).
¹H-NMR (300 MHz, 20°C, CDCl₃):
δ = 8.2 (2H, m, H_{Ar, Nitro}), 7.66 (2H, m, H_{Ar, Nitro}), 7.56 (2H, m, H_{Ar}), 7.39 (2H, m, H_{Ar}); ¹³C{1H}-NMR (75.4 MHz, 20°C, CDCl₃): δ = 147,0 (S, C-NO₂), 132.6 (s, C_{Ar}), 132.3 (s, C_{Ar}), 131,9 (s, C_{Ar}), 130,3 (s, C_{Ar}), 129,3 (S, C_{Ar}), 128.6 (s, C_{Ar}), 125,0 (s, C_{Ar}), 123,7 (s, C_{Ar}), 94,8 (s, C_{Acetylen}).
Auf gleiche Weise werden hergestellt (Ausbeute in [%]):
1-(4-Acetylphenyl)-2-phenylethin (99)
1-(4-Nitrophenyl)-2-phenylethin (99)
1-(4-Chlorphenyl)-2-phenylethin (90)
1-(4-n-Butylphenyl)-2-phenylethin (80)
1-(4-Methoxyphenyl)-2-phenylethin (80)
3-(4-Acetylphenyl)-1-trimethylsiloxy-prop-2-in (38)
3-(4-Acetylphenyl)-1-tetrahydropyranyl-prop-2-in (30)
3-(4-Chlorphenyl)-1-trimethylsiloxy-prop-2-in
3-(4-Chlorphenyl)-1-tetrahydropyranyl-prop-2-in (40)
1-(2-Pyridyl)-2-phenylethin (30)
4-(6-Methoxynaphthyl)-1-methyl-but-2-in-1-ol (68)

### Beispiel 14: in situ präparierter Katalysator

2,0 g (10 mmol 4-Bromacetophenon und 1,2 g (12 mmol) Phenylacetylen werden mit 10 g (0,1 mol %) Palladiumacetat und xx mg (0,1 mol %) Tri-o-tolylphosphin in 30 ml Triethylamin 4 Stunden auf 90°C erhitzt. Die Reaktionslösung wird heiß filtriert, um das quantitativ ausgefallene Triethylammoniumbromid abzutrennen und das Produkt mittels fraktionierter Kristallisation abgetrennt.
Ausbeute: 93 % 1-(4-Acetylphenyl)-2-phenylethin.

## Patentansprüche

1. Verfahren zur Herstellung von aromatischen Acetylenen der Formel (I)
(I) Ar-C≡C-R^{8a}
worin Ar für 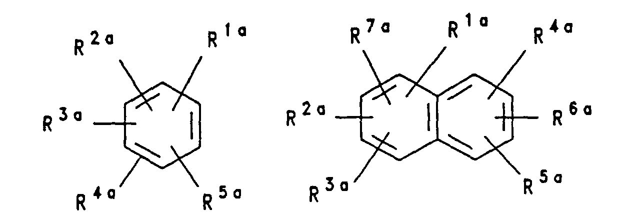 steht und
R^{1a} bis R^{7a} unabhängig voneinander Wasserstoff, C₁-C₈-Alkyl, Alkoxy-(C₁-C₈), Acyloxy-(C₁-C₈), OPhenyl, Phenyl, Fluor, Chlor, Brom, OH, NO₂, CN, COOH, CHO, SO₃H, SO₂R, SOR, NH₂, NHAlkyl-(C₁-C₁₂), N-Alkyl₂-(C₁-C₁₂), C-Hal₃, NHCO-Alkyl-(C₁-C₈), CO-Alkyl-(C₁-C₈), COO-Alkyl-(C₁-C₁₂), CONH₂, CO-Alkyl-(C₁-C₁₂), NHCOH, NCOO-Alkyl-(C₁-C₈), CO-Phenyl, COO-Phenyl, CHCHCO₂-Alkyl-(C₁-C₁₂), CHCHCO₂H, PO-Phenyl₂, PO-Alkyl₂-(C₁-C₈), 5-Ring-Heteroaryl, 6-Ring-Heteroaryl bedeuten, wobei R=(C₁-C₈)Alkyl oder Pheny ist und R^{8a} für H, C₁-C₁₂-Alkyl, Si(CH₃)₃, CH₂OH, C(CH₃)₂OH oder Ar steht, durch Umsetzung von Halogenaromaten oder Arylsulfonaten der Formel (II)
Ar-X (II)
mit monosubstituierten Acetylenen der Formel (III)
H―C≡C―R^{8a} (III)
worin Ar und R^{8a} die angegebene Bedeutung besitzen und X für Brom oder Chlor oder OSO₂R steht, **dadurch gekennzeichnet, daß** man als Katalysator eine Palladiumverbindung der allgemeinen Formel (IV) 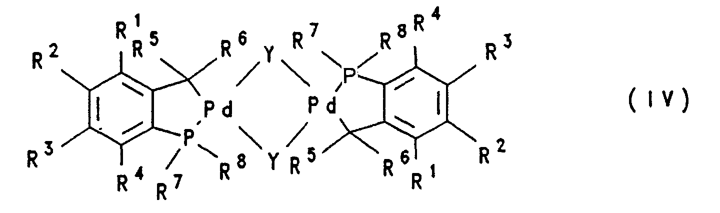 worin
R¹, R², R³, R⁴, R⁵, R⁶ unabhängig voneinander Wasserstoff, (C₁-C₄)-Alkyl, (C₁-C₄)-Alkoxy, Fluor, NH₂, NH-Alkyl-(C₁-C₄), N(Alkyl)₂-(C₁-C₄), CO₂-Alkyl-(C₁-C₄), OCO-Alkyl-(C₁-C₄), oder Phenyl bedeuten,
oder R¹ und R², R² und R³, R³ und R⁴, R⁵ und R⁶ zusammen einen aliphatischen oder aromatischen Ring bilden, und
R⁷, R⁸ (C₁-C₈)-Alkyl, (C₃-C₁₂)-Cycloalkyl, substituiertes oder unsubstituiertes Aryl sind und Y ein Anion einer anorganischen oder organischen Säure bedeutet, einsetzt.

2. Verfahren nach Anspruch 1, **dadurch gekennzeichnet, daß** in Formel (IV) R¹ bis R⁶ unabhängig voneinander Wasserstoff, Alkyl-(C₁-C₄), Phenyl, Cycloalkyl-(C₅-C₈), R⁷ und R⁸ Phenyl, Tolyl, Xylyl, Mesityl, Alkyl-(C₁-C₈) und Cycloalkyl-(C₅-C₈) bedeuten und Y für Acetat, Propionat, Benzoat, Chlorid, Bromid, lodid, Fluorid, Sulfat, Hydrogensulfat, Nitrat, Phosphat, Tetrafluoroborat, Tosylat, Mesylat, Acetylacetonat, Hexafluoracetylacetonat oder Pyrazolyl steht.

3. Verfahren nach Anspruch 1, **dadurch gekennzeichnet, daß** Formel (IV) R¹ bis R⁶ für H, Alkyl, Phenyl, R⁷, R⁸ für Phenyl, Tolyl, Xylyl, Mesityl, und Alkyl stehen.

4. Verfahren nach Anspruch 1, **dadurch gekennzeichnet, daß** als Katalysator die Verbindungen
trans-Di-*µ*-acetato-bis[o-di-tolylphosphino)benzyl]dipalladium(II)
trans-Di-*µ*-chloro-bis[di-o-tolylphosphino)benzyl]dipalladium(II)
trans-Di-*µ*-bromo-bis[o-di-o-tolylphosphino)benzyl]dipalladium(II)
trans-Di-*µ*-iodo-bis[o-(di-o-tolylphosphino)benzyl]dipalladium(II)
trans-Di-*µ*-acetato-bis[o-(dimesitylphosphino)-3,5-dimethylbenzyl]-dipalladium(II)
trans-Di-*µ*-chloro-bis[o-(dimesitylphosphino)-3,5-dimethylbenzyl]-dipalladium(II)
trans-Di-*µ* -bromo-bis[o-(dimesitylphosphino)-3,5-dimethylbenzyl]-dipalladium(II)
trans-Di-*µ*-iodo-bis[o-(dimesitylphosphino)-3,5-dimethylbenzyl]-dipalladium(II)
eingesetzt werden.

5. Verfahren nach mindestens einem der Ansprüche 1 bis 4, **dadurch gekennzeichnet, daß** der Katalysator in situ hergestellt wird.

6. Verfahren nach mindestens einem der Ansprüche 1 bis 5, **dadurch gekennzeichnet, daß** R^{1a} bis R^{7a} in Formel (I) Wasserstoff, C₁-C₄-Alkyl, Alkoxy-(C₁-C₄), Acyloxy-(C₁-C₄), OPhenyl, Phenyl, Fluor, Chlor, OH, NO₂, CN, COOH, CHO, NH₂, NHAlkyl-(C₁-C₆), N-Alkyl₂-(C₁-C₆), NHCO-Alkyl-(C₁-C₄), CO-Alkyl-(C₁-C₄), COO-Alkyl-(C₁-C₆), CONH₂, CO-Alkyl-(C₁-C₆), NHCOH, NCOO-Alkyl-(C₁-C₄), CHCHCO₂-Alkyl-(C₁-C₆), CHCHCO₂H, 5-Ring-Heteroaryl, 6-Ring-Heteroaryl bedeutet.

7. Verfahren nach mindestens einem der Ansprüche 1 bis 6, **dadurch gekennzeichnet, daß** 3, insbesondere 4 der Substituenten R^{1a} bis R^{7a} Wasserstoff darstellen.

8. Verfahren nach mindestens einem der Ansprüche 1 bis 6, **dadurch gekennzeichnet, daß** Formel (I) für 1-(4-Nitrophenyl)-2-phenylethin, 1-(4-Acetylphenyl)-2-phenylethin, 2-(6-Methoxynaphthyl)-1-trimethyl-silylethin, 3-(6-Methoxynaphthyl)-1-methyl-propargylalkohol, (4-lsobutylphenyl)-1-ethin, 6-Methoxynaphthyl-2-ethin, 4-Hydroxyphenyl-1-ethin und 4-(2-Hydroxyethoxy)phenyl-1-ethin steht.

9. Verfahren nach mindestens einem der Ansprüche 1 bis 8, **dadurch gekennzeichnet, daß** dipolar aprotische und polare Lösungsmittel, bevorzugt Trialkylamine, insbesondere Triethylamin eingesetzt werden.

10. Verfahren nach mindestens einem der Ansprüche 1 bis 9, **dadurch gekennzeichnet, daß** die bei der Reaktion entstehende Säure HX durch Zusatz von Base, insbesondere eines Amins abgefangen wird.

11. Verfahren nach Anspruch 10, **dadurch gekennzeichnet, daß** als Base Alkylamine, insbesondere Triethylamin eingesetzt werden.

12. Verfahren nach mindestens einem der Ansprüche 1 bis 11, **dadurch gekennzeichnet, daß** die Reaktion bei Temperaturen von 20 bis 150°C, insbesondere 50 bis 120°C, bevorzugt 60 bis 110°C durchgeführt wird.

## Claims

1. A process for preparing aromatic acetylenes of the formula (I)
(I) Ar-C≡C-R^{8a}
where Ar is 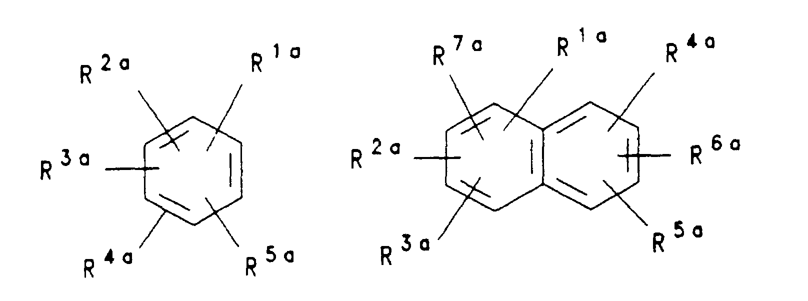 and
R^{1a} to R^{7a} are, independently of one another, hydrogen, C₁-C₈-alkyl, alkoxy-(C₁-C₈), acyloxy-(C₁-C₈), O-phenyl, phenyl, fluorine, chlorine, bromine, OH, NO₂, CN, COOH, CHO, SO₃H, SO₂R, SOR, NH₂, NH-alkyl-(C₁-C₁₂), N-alkyl₂- (C₁-C₁₂), C-Hal₃, NHCO-alkyl-(C₁-C₈), CO-alkyl-(C₁-C₈), COO-alkyl-(C₁-C₁₂), CONH₂, CO-alkyl-(C₁-C₁₂), NHCOH, NCOO-alkyl-(C₁-C₈), CO-phenyl, COO-phenyl, CHCHCO₂-alkyl-(C₁-C₁₂), CHCHCO₂H, PO-phenyl₂, PO-alkyl₂-(C₁-C₈), 5-membered ring heteroaryl or 6-membered ring heteroaryl, where R=(C₁-C₈)alkyl or phenyl, and R^{8a} is H, C₁-C₁₂-alkyl, Si(CH₃)₃, CH₂OH, C(CH₃)₂OH or Ar, by reacting haloaromatics or aryl sulfonates of the formula (II)
Ar-X (II)
with monosubstituted acetylenes of the formula (III)
H-C≡C-R^{8a} (III)
where Ar and R^{8a} areas defined above and X is bromine or chlorine or OSO₂R, wherein a palladium compound of the formula (IV) 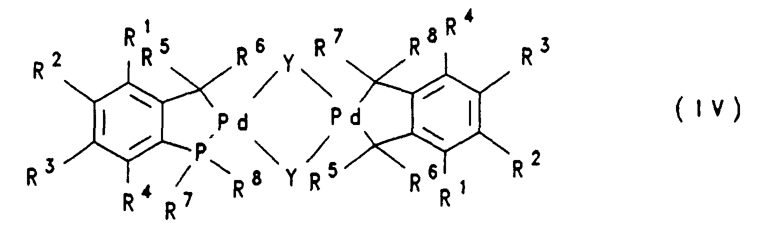 where
R¹, R², R³, R⁴, R⁵, R⁶ are, independently of one another, hydrogen, (C₁-C₄)-alkyl, (C₁-C₄)-alkoxy, fluorine, NH₂, NH-alkyl-(C₁-C₄), N(alkyl)₂-(C₁-C₄), CO₂-alkyl-(C₁-C₄), OCO-alkyl-(C₁-C₄) or phenyl,
or R¹ and R², R² and R³, R³ and R⁴, R⁵ and R⁶ together form an aliphatic or aromatic ring, and
R⁷, R⁸ are (C₁-C₈)-alkyl, (C₃-C₁₂)-cycloalkyl, substituted or unsubstituted aryl and Y is an anion of an inorganic or organic acid, is used as catalyst.

2. The process as claimed in claim 1, wherein, in formula (IV), R¹ to R⁶ are, independently of one another, hydrogen, alkyl-(C₁-C₄), phenyl, cyclo-alkyl-(C₅-C₈), R⁷ and R⁸ are phenyl, tolyl, xylyl, mesityl, alkyl-(C₁-C₈) and cycloalkyl-(C₅-C₈) and Y is acetate, propionate, benzoate, chloride, bromide, iodide, fluoride, sulfate, hydrogensulfate, nitrate, phosphate, tetrafluoroborate, tosylate, mesylate, acetylacetonate, hexafluoro-acetylacetonate, or pyrazolyl.

3. The process as claimed in claim 1, wherein, in formula (IV), R¹ to R⁶ are H, alkyl, phenyl, R⁷, R⁸ are phenyl, tolyl, xylyl, mesityl and alkyl.

4. The process as claimed in claim 1, wherein the compounds
trans-di-µ-acetato-bis[o-(di-o-tolylphosphino)-benzyl]dipalladium(II)
trans-di-p-chloro-bis[o-(di-o-tolylphosphino)-benzyl]dipalladium(II)
trans-di-µ-bromo-bis[o-(di-o-tolylphosphino)benzyl]dipalladium(II)
trans-di-µ-iodo-bis[o-(di-o-tolylphosphino)benzyl]-dipalladium(II)
trans-di-µ-acetato-bis[o-(dimesitylphosphino)-3,5-dimethylbenzyl]dipalladium(II)
trans-di-µ-chloro-bis[o-(dimesitylphosphino)-3,5-dimethylbenzyl]dipalladium(II)
trans-di-µ-bromo-bis[o-(dimesitylphosphino)-3,5-dimethylbenzyl]dipalladium(II)
trans-di-µ-iodo-bis[o-(dimesitylphosphino)-3,5-dimethylbenzyl]dipalladium(II)
are used as catalyst.

5. The process as claimed in at least one of claims 1 to 4, wherein the catalyst is prepared in situ.

6. The process as claimed in at least one of claims 1 to 5, wherein R^{1a} to R^{7a} in formula (I) are hydrogen, (C₁-C₄)-alkyl, alkoxy-(C₁-C₄), acyloxy-(C₁-C₄), 0-phenyl, phenyl, fluorine, chlorine, OH, NO₂, CN, COOH, CHO, NH₂, NH-alkyl-(C₁-C₆), N-alkyl₂-(C₁-C₆), NHCO-alkyl-(C₁-C₄), CO-alkyl-(C₁-C₄), COO-alkyl-(C₁-C₆), CONH₂, CO-alkyl-(C₁-C₆), NHCOH, NCOO-alkyl-(C₁-C₄), CHCHCO₂-alkyl-(C₁-C₆), CHCHCO₂H, 5-membered ring heteroaryl or 6-membered ring heteroaryl.

7. The process as claimed in at least one of claims 1 to 6, wherein 3, in particular 4, of the substituents R^{1a} to R^{7a} are hydrogen.

8. The process as claimed in at least one of claims 1 to 6, wherein formula (I) represents 1-(4-nitro-phenyl)-2-phenylethyne, 1-(4-acetylphenyl)-2-phenylethyne, 2-(6-methoxynaphthyl)-1-trimethyl-silylethyne, 3-(6-methoxynaphthyl)-1-methyl-propargyl alcohol, (4-isobutylphenyl)-1-ethyne, 6-methoxynaphthyl-2-ethyne, 4-hydroxyphenyl-1-ethyne and 4-(2-hydroxyethoxy)phenyl-1-ethyne.

9. The process as claimed in at least one of claims 1 to 8, wherein dipolar aprotic and polar solvents, preferably trialkylamines, in particular triethylamine, are used.

10. The process as claimed in at least one of claims 1 to 9, wherein the acid HX formed during the reaction is neutralized by addition of base, in particular an amine.

11. The process as claimed in claim 10, wherein alkylamines, in particular triethylamine, are used as base.

12. The process as claimed in at least one of claims 1 to 11, wherein the reaction is carried out at temperatures of from 20 to 150°C, in particular from 50 to 120°C, preferably from 60 to 110°C.

## Revendications

1. Procédé de préparation d'acétylènes aromatiques de formule (I)
Ar-C≡C-R^{8a} (I)
dans laquelle Ar représente 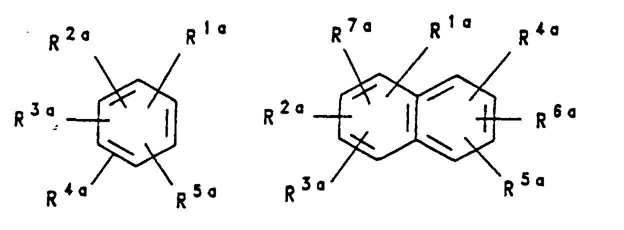 et
R^{1a} à R^{7a} représentent chacun indépendamment des autres un atome d'hydrogène, un groupe alkyle en C₁-C₈, alcoxy en C₁-C₈, acyloxy en C₁-C₈, O-phényle, phényle, fluoro, chloro, bromo, OH, NO₂, CN, COOH, CHO, SO₃H, SO₂R, SOR, NH₂, NH-alkyle en C₁-C₁₂, N-(alkyle en C₁-C₁₂)₂, C-Hal₃, NHCO-(alkyle en C₁-C₈), CO-(alkyle en C₁-C₈), COO-(alkyle en C₁-C₁₂), CONH₂, CO-(alkyle en C₁-C₁₂), NHCOH, NCOO-(alkyle en C₁-C₈), CO-phényle, COO-phényle, CHCHCO₂-(alkyle en C₁-C₁₂), CHCHCO₂H, PO-phényl₂, PO-(alkyle en C₁-C₈)₂, hétéroaryle pentacyclique, hétéroaryle hexacyclique, où R est un groupe alkyle en C₁-C₈ ou phényle, et R^{8a} est H, un groupe alkyle en C₁-C₁₂, Si(CH₃)₃, CH₂OH, C(CH₃)₂OH ou Ar, par réaction de composés aromatiques halogénés ou d'arylsulfonates de formule (II)
Ar-X (II)
avec des acétylènes monosubstitués de formule (III)
H-C≡C-R^{8a} (III)
dans lesquelles Ar et R^{8a} ont les significations indiquées, et X est le brome ou le chlore ou OSO₂R, **caractérisé en ce qu'**on utilise en tant que catalyseur un composé du palladium de formule générale (IV) 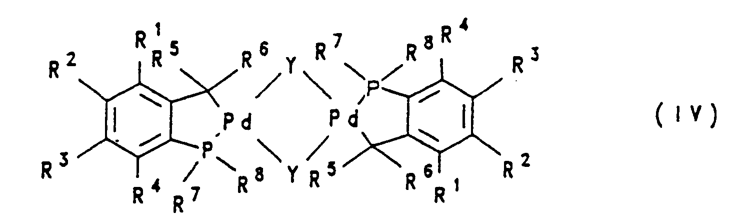 dans laquelle
R¹, R², R³, R⁴, R⁵ et R⁶ représentent chacun indépendamment des autres un atome d'hydrogène, un groupe alkyle en C₁-C₄, alcoxy en C₁-C₄, fluoro, NH₂, NH-(alkyle en C₁-C₄), N-(alkyle en C₁-C₄)₂, CO₂-(alkyle en C₁-C₄), OCO-(alkyle en C₁-C₄) ou phényle, ou encore R¹ et R², R² et R³, R³ et R⁴, R⁵ et R⁶, forment ensemble un noyau aliphatique ou aromatique, et
R⁷ et R⁸ sont des groupes alkyle en C₁-C₈, cycloalkyle en C₃-C₁₂, aryle non-substitué ou substitué, et Y est un anion d'un acide inorganique ou organique.

2. Procédé selon la revendication 1, **caractérisé en ce que**, dans la formule (IV), R¹ à R⁶ représentent chacun indépendamment des autres un atome d'hydrogène, un groupe alkyle en C₁-C₄, phényle, cycloalkyle en C₅-C₈, R⁷ et R⁸ représentent chacun le groupe phényle, tolyle, xylyle, mésityle, un groupe alkyle en C₁-C₈ ou cycloalkyle en C₅-C₈, et Y représente le groupe acétate, propionate, benzoate, chlorure, bromure, iodure, fluorure, sulfate, hydrogénosulfate, nitrate, phosphate, tétrafluoroborate, tosylate, mésylate, acétylacétonate, hexafluoracétyl-acétonate ou pyrazolyle.

3. Procédé selon la revendication 1, **caractérisé en ce que**, dans la formule (IV), R¹ à R⁶ sont H ou des groupes alkyle ou phényle, R⁷ et R⁸ sont des groupes phényle, tolyle, xylyle, mésityle ou alkyle.

4. Procédé selon la revendication 1, **caractérisé en ce qu'**on utilise en tant que catalyseur les composés suivants :
trans-di-*µ*-acétato-bis[o-di-tolylphosphino)benzyl]-dipalladium(II)
trans-di-*µ*-chloro-bis[di-o-tolylphosphino)benzyl]-dipalladium(II)
trans-di-*µ*-bromo-bis[o-di-o-tolylphosphino)benzyl]-dipalladium(II)
trans-di-*µ*-iodo-bis[o-di-o-tolylphosphino)benzyl]-dipalladium(II)
trans-di-*µ*-acétato-bis[o-dimésitylphosphino)-3,5-diméthylbenzyl]dipalladium(II)
trans-di-*µ*-chloro-bis[o-dimésitylphosphino)-3,5-diméthylbenzyl]dipalladium(II)
trans-di-*µ*-bromo-bis[o-dimésitylphosphino)-3,5-diméthylbenzyl]dipalladium(II)
trans-di-*µ*-iodo-bis[o-dimésitylphosphino)-3,5-diméthylbenzyl]dipalladium(II).

5. Procédé selon au moins l'une des revendications 1 à 4, **caractérisé en ce que** le catalyseur est préparé in situ.

6. Procédé selon au moins l'une des revendications 1 à 4, **caractérisé en ce que** R^{1a} à R^{7a}, dans la formule (I), représentent chacun un atome d'hydrogène, un groupe alkyle en C₁-C₄, alcoxy en C₁-C₄, acyloxy en C₁-C₄, O-phényle, phényle, fluoro, chloro, OH, NO₂, CN, COOH, CHO, NH₂, NH-alkyle en C₁-C₆, N-(alkyle en C₁-C₆)₂, NHCO-(alkyle en C₁-C₄), CO-(alkyle en C₁-C₄), COO-(alkyle en C₁-C₆), CONH₂, CO-(alkyle en C₁-C₆), NHCOH, NCOO- (alkyle en C₁-C₄), CHCHCO₂-(alkyle en C₁-C₆), CHCHCO₂H, hétéroaryle penta-cyclique, hétéroaryle hexacyclique.

7. Procédé selon au moins l'une des revendications 1 à 6, **caractérisé en ce que** 3 et en particulier 4 des substituants R^{1a} à R^{7a} sont des atomes d'hydrogène.

8. Procédé selon au moins l'une des revendications 1 à 6, **caractérisé en ce que** la formule (I) représente le 1- (4-nitrophényl)-2-phényléthyne, le 1-(4-acétylphényl)-2-phényléthyne, le 2-(6-méthoxynaphtyl)-1-triméthyl-silyléthyne, l'alcool 3-(6-méthoxynaphtyl)-1-méthyl-propargylique, le (4-isobutylphényl)-1-éthyne, le 6-méthoxynaphtyl-2-éthyne, le 4-hydroxyphényl-1-éthyne, et le 4-(2-hydroxyéthoxy)phényl-1-éthyne.

9. Procédé selon au moins l'une des revendications 1 à 8, **caractérisé en ce qu'**on utilise des solvants dipolaires aprotiques et polaires, de préférence des trialkyl-amines, en particulier la triéthylamine.

10. Procédé selon au moins l'une des revendications 1 à 9, **caractérisé en ce que** l'acide HX qui se forme lors de la réaction est fixé par addition d'une base, en particulier d'une amine.

11. Procédé selon la revendication 10, **caractérisé en ce qu'**on utilise en tant que base des alkylamines, en particulier la triéthylamine.

12. Procédé selon au moins l'une des revendications 1 à 11, **caractérisé en ce que** la réaction est mise en oeuvre à des températures de 20 à 150°C, en particulier de 50 à 120°C, de préférence de 60 à 110°C.
